**Europäisches Patentamt**

**European Patent Office**

**Office européen des brevets**

(19)

(11) Publication number: **0 099 665**
**B1**

(12)
# EUROPEAN PATENT SPECIFICATION

(45) Date of publication of patent specification: **26.08.87**

(21) Application number: **83303723.7**

(22) Date of filing: **28.06.83**

(51) Int. Cl.⁴: **C 07 C 51/12,** C 07 C 45/49,
C 07 C 29/32, C 07 C 67/36,
C 07 C 53/08, C 07 C 31/08,
C 07 C 47/06, C 07 C 69/14,
B 01 J 37/20

(54) **Catalytic carbonylation of alcohols.**

(30) Priority: **30.06.82 US 393931**
**30.06.82 US 393935**

(43) Date of publication of application:
**01.02.84 Bulletin 84/05**

(45) Publication of the grant of the patent:
**26.08.87 Bulletin 87/35**

(84) Designated Contracting States:
**BE DE FR GB NL**

(56) References cited:
**DE-B-2 824 125**
**DE-C- 922 231**
**GB-A-2 048 267**
**GB-A-2 089 803**

(73) Proprietor: **CHEVRON RESEARCH COMPANY**
**525 Market Street**
**San Francisco California 94105 (US)**

(72) Inventor: **Current, Steven P.**
**1207 Ridgeview Heights**
**Novato, CA 94947 (US)**

(74) Representative: **Kosmin, Gerald Emmanuel et al**
**HASELTINE, LAKE & CO. Hazlitt House 28**
**Southampton Buildings Chancery Lane**
**London, WC2A 1AT (GB)**

**The file contains technical information
submitted after the application was filed and
not included in this specification**

Courier Press, Leamington Spa, England.

## Description

This invention relates to a process for the catalytic carbonylation of alcohols and is concerned with a process for converting alcohols into oxygen-containing products having at least one more carbon atom than the starting alcohol. By virtue of the process of the present invention, it is possible to obtain carboxylic acids, alcohols, or aldehydes, or the secondary products thereof (which may be formed in situ by a subsequent reaction) by reaction of an alcohol with hydrogen and carbon monoxide in the presence of a heterogeneous sulphided catalyst.

U.S. Patent No. 2,623,906 discloses that at pressures above 1,000 atmospheres and in the presence of a cobalt catalyst, primary, secondary or tertiary alcohols react with synthesis gas (a mixture of hydrogen and carbon monoxide) to form glycol ethers and monohydric alcohols containing at least one more carbon atom per molecule than the original alcohol reactant.

U.S. Patent No. 3,285,948 discloses that an improved yield of ethanol from methanol can be obtained by conducting the synthesis gas homologation reaction in the presence of a cobalt catalyst which is promoted with iodine and a metal halide selected from ruthenium halide and osmium halide.

U.S. Patent No. 4,111,837 discloses a process for producing ethanol which comprises reacting methanol with carbon monoxide and hydrogen in the presence of a catalyst consisting essentially of a methanol-soluble cobalt carbonyl and methanol-insoluble rhenium metal.

U.S. Patent No. 4,304,946 discloses a process for producing ethanol from methanol, carbon monoxide and hydrogen which comprises conducting the reaction in the presence of a cobalt sulphide or a mixture of a cobalt sulphide and at least one of a nitrogen-containing compound and a phosphorus compound.

It has now been found, in accordance with the present invention, that alcohols can be converted into useful oxygenated products having at least one more carbon atom than the starting alcohol in improved yield and selectivity by utilizing an insoluble heterogeneous sulphided nickel catalyst system. An advantage of this process is that the insoluble heterogeneous catalyst employed is much easier to separate from the reaction products that the soluble homogeneous catalysts employed in the prior art processes.

In addition, it has been found that the process of the present invention does not require the use of any soluble catalyst promoters. This is particularly advantageous, since the absence of a halide promoter in the system obviates the need for expensive corrosion resistant equipment.

Thus in accordance with the present invention, there is provided a process for catalytically converting an alcohol into at least one oxygen-containing product, such as a carboxylic acid, alcohol, aldehyde or a secondary product thereof, having at least one more carbon atom than the starting alcohol, characterised in that an alcohol having from one to twenty carbon atoms is reacted with hydrogen and carbon monoxide at a temperature in the range from 150 to 350°C and a pressure in the range from 500 to 5,000 psig (34 to 345 bar) in the presence of an insoluble heterogeneous sulphided catalyst comprising nickel, or nickel in admixture with a co-catalyst selected from the elements of Group VB, Group VIB and the Actinide series of the Periodic Table, the nickel and co-catalyst when present being in sulphide form, to obtain the desired reaction product.

Oxygen-containing carbon compounds which can be obtained with high selectivity in the process of the invention include carboxylic acids, alcohols, aldehydes or the secondary products which may be formed therefrom under the reaction conditions in a subsequent reaction, for example, esterification, condensation or dehydration.

Illustrative of a typical batch procedure, in the process of the invention the alcohol is charged to a high pressure reactor, and then there is introduced a heterogeneous sulphided catalyst system comprising nickel or nickel plus an element of Group VB, Group VIB or the Actinide series. The reactor is pressurized with a mixture containing carbon monoxide and hydrogen and heated for a suitable length of time to give the desired conversion. Liquid and gaseous products and reactants can be easily separated from the catalyst by filtration, distillation or other methods. Unreacted starting materials can be recycled. The products can be isolated by means of one of a number of known methods, including distillation. In some cases it may be advantageous to further process the products. For example, methyl acetate can be easily hydrolyzed to acetic acid.

The process of the present invention can also be run in a continuous fashion. This is particularly advantageous as the catalyst is not soluble in the reaction medium. A number of reactor configurations are suitable including fixed or fluid beds, slurry beds or stirred tank reactors. As with a batch reaction, unreacted starting materials can be easily recycled and, if desired, the products can be further processed.

The alcohols suitable for use in the present invention may be primary, secondary, tertiary or benzylic alcohols having from one to twenty carbon atoms. Diols and polyols may also be used. A preferred alcohol is methanol. If desired, the reactant alcohol may be diluted with an alcohol-miscible solvent such as dioxan, tetrahydrofuran, or N-methylpyrrolidinone. When methanol is used as the starting alcohol, reaction products typically formed include acetic acid, ethanol, acetaldehyde or methyl acetate.

The heterogeneous sulphided catalyst system employed in the present process comprises nickel sulphide alone or a composite of sulphides of a nickel component and a Group VB, Group VIB or Actinide component. Cocatalysts suitable for admixture with the nickel component include tantalum, chromium, vanadium, thorium and tungsten. A particularly preferred catalyst system comprises nickel and molybdenum. The catalyst system may optionally contain phosphorus or silicon.

In carrying out the reaction, it is usually desirable, although not essential, to place the catalyst on a support. Generally, the support should be a solid, inert material which is relatively insoluble in the solvent employed. Suitable supports include various treated or untreated organic and inorganic supports. Included among these are synthetic and naturally occurring polymers, alumina, silica, titania, silica-alumina, zeolites, glass and carbon. Particularly preferred supports are alumina and silica-alumina.

The metals may be added to the support using a method known in the art, such as by impregnation or co-precipitation. The method of loading the catalyst on the support will depend on the nature and composition of the support. Generally, the most convenient method of depositing the metals on the support is by adding a solution of metal salts to the support and subsequently converting them to an insoluble form.

An especially suitable catalyst precursor may be prepared by impregnating alumina with an aqueous or organic solution of the metal salts, either together or sequentially, followed by drying and calcining to give the metal oxides.

The catalyst may be converted to its active sulphide form by any of a number of conventional procedures. Treatment with hydrogen sulphide or other sulphur-containing compound such as carbon sulphide, dimethyl disulphide or sulphur, in the presence of hydrogen or synthesis gas is effective. This treatment can be either prior to or concurrent with the alcohol carbonylation reaction.

In the process of the present invention alcohols are reacted with carbon monoxide and hydrogen (synthesis gas). Synthesis gas produced by the reaction of carbonaceous material with water is suitable. Mixtures of, for example, carbon dioxide and hydrogen, or carbon monoxide and water, may also be employed. Whether introduced originally, or produced *in situ* under processing conditions, carbon monoxide and hydrogen are required for the reaction.

The relative molar quantities of carbon monoxide and hydrogen present during the reaction can vary in the range from 10:1 to 1:10, and preferably in the range from 3:1 to 1:3. An inert diluent gas such as nitrogen or helium may be included if desired.

The carbonylation reaction requires a relatively high pressure for optimum selectivity and yield of product. The pressure should be maintained in the range from 500 to 5,000 psig (34 to 345 bar), and preferably in the range from 800 to 2000 psig (52 to 138 bar).

The reaction is conduced at a temperature in the range from 150 to 350°C, and preferably in the range from 190 to 290°C.

The time that the reactants are in contact with the catalyst will be dependent, among other, factors, on the temperature, pressure, alcohol reactant, catalyst, reactor configuration and the desired level of conversion.

The solid catalyst can be easily separated from the generally liquid and gaseous reaction products and unreacted starting materials by, for example, filtration, centrifugation, settling out or distillation. The catalyst can be reused in a subsequent reaction. Unreacted starting materials can be separated from reaction products and are suitable for recycle in the process.

The products of the reaction, which can be isolated by one of a number of well-known methods, such as distillation, are generally useful as solvents or chemical intermediates. In some cases it may be advantageous to further process the reaction products by well-known means to form other useful products. For example, methyl acetate can be hydrolyzed to acetic acid, and ethanol and phenethyl alcohol can be dehydrated to ethylene and styrene, respectively.

The following Examples are provided to illustrate the invention. Examples 1 to 3 and 14 illustrate the preparation of non-sulphided catalysts suitable for use in the invention, whilst the remainder illustrate the process of the invention.

Example 1

A catalyst was prepared from nickel nitrate hexahydrate (8.1 g) and commercial phosphomolybdic acid (15.1 g) by dissolving in water (50 ml), evaporating to dryness on a hot-plate, and further heating first at 200°C for 2 hours and finally at 325°C for 2 hours to yield 13.4 g of a dark brown solid. This catalyst was used in Example 4.

Example 2

A catalyst was prepared by impregnating 50 g of alumina with a water solution (32 ml) of nickel nitrate hexahydrate (8.6 g) and silicotungstic acid hydrate (10.3 g). The catalyst was dried under vacuum at 120°C and then calcined in air at 475°C for 4 hours. This catalyst was used in Example 8-g.

Example 3

A catalyst was prepared by impregnating 50 g of a commercial vanadium oxide on alumina catalyst (Girdler V-0301) with a water solution (32 ml) of nickel nitrate hexahydrate (7.8 g). The catalyst was dried at 120°C and then calcined at 475°C for 4 hours. This catalyst was used in Example 8-e.

Example 4

A 300 ml stainless steel autoclave was charged with 75 ml of methanol and 5.0 g of an unsupported catalyst comprising nickel, molybdenum and phosphorus oxides (as described in Example 1) that had been

3

treated with 10% hydrogen sulphide in hydrogen at 325°C followed by hydrogen at 400°C. The reactor was sealed and pressurized to 1000 psi (68 bar) with a mixture of one part hydrogen and two parts carbon monoxide, and then heated to 235°C. After 6.0 hours the reactor was cooled and the pressure released. Analysis of the liquid products by gas chromatography indicated methyl acetate (121 mmol) and ethanol (12.8 mmol) as major products as well as minor amounts of n-propanol and acetic acid.

Example 5

A 300 ml stainless steel autoclave was charged with 10.00 g of a catalyst comprising nickel, molybdenum and phosphorus (approximately 3, 14 and less than 1 wt. %, respectively) oxides on alumina, 2.50 g of sulphur and 50 ml of methanol. The reactor was sealed, flushed with nitrogen, and pressurized to 900 psi with a mixture of two parts hydrogen and one part carbon monoxide. The reactor was heated to 250°C and the pressure adjusted to 2500 psi (172 bar). After 4 hours, the reactor was cooled with ice and the pressure released. Analysis of the liquid product by gas chromatography, after addition of 1,4-dioxan as internal standard, indicated methyl acetate (27.3 mmol) and ethanol (12.4 mmol) as products as well as methyl ether and methyl sulphide.

Example 6

A 300 ml stainless steel autoclave was purged with nitrogen and charged with 100 ml of methanol, 1,4-dioxan (as internal standard), and 5.0 g of a catalyst comprising nickel, molybdenum and phosphorus (approximately 6, 15 and 2 wt%, respectively) oxides on silica-alumina that had been previously treated with 10% hydrogen sulphide in hydrogen at 325°C. The autoclave was sealed and pressurized with 350 psi of a mixture of two parts hydrogen and one part carbon monoxide, then heated to 240°C to give a final pressure of 1200 psi. After 2 hours, analysis of a small sample indicated methyl acetate (7.8 mmol) and ethanol (0.25 mmol) as major products.

This example was repeated except that the initial pressure was 1000 psi and the final pressure was 2300 psi. In this case, the products after two hours of reaction were methyl acetate (34.0 mmol) and ethanol (7.8 mmol).

Example 7

Four 18 ml stainless steel reactors were each charged with 5 ml of methanol and 0.50 g of a catalyst comprising nickel, molybdenum and phosphorus (approximately 3, 13 and 2 wt.%, respectively) oxides on alumina. Reactors 1—3 each contained a source of sulphur as indicated in the following Table 1. The reactors were sealed and pressurized to 850 psi with a mixture of 2 parts hydrogen and 1 part carbon monoxide. The reactors were heated at 240°C with gentle shaking for 4.0 hours. After cooling and venting, the contents of the reactors were analyzed by gas chromatography. The results are indicated in Table 1. In addition to the indicated products, methyl ether and methyl sulphide were also produced.

TABLE 1

| Charges | Products | | |
|---|---|---|---|
| Sulphur compound | Amount | Methyl acetate | Ethanol |
| 1. Methyl disulphide | 0.7 mmol | 0.2 mmol | trace |
| 2. Carbon disulphide | 1.2 | 1.2 | 0.3 mmol |
| 3. Sulphur | 1.6 | 0.4 | 0.1 |
| 4. None | 0 | trace | 0 |

Example 8

A series of 18 ml stainless steel reactors were each charged with methanol (5 ml), 1,4-dioxan (0.10 ml), sulphur (0.13 g) and catalyst (0.50 g) as indicated in the following Table 2. The reactors were sealed and pressurized with 900 psi of a mixture of two parts hydrogen and one part carbon monoxide. The reactors were heated at 240°C with gentle shaking for 4.0 hours. After cooling and venting, the contents were analyzed by gas chromatography using 1,4-dioxan as internal standard. In addition to the products indicated, methyl ether and methyl sulphide were also formed.

TABLE 2

| | Catalyst[1] | Products | |
|---|---|---|---|
| | | Methyl acetate | Ethanol |
| a. | Ni(8), W(19), Ti(8) on silica-alumina | 3.6 mmol | 0.3 mmol |
| b. | Ni(3), Mo(12), P(0.3) on silica | 2.1 | 0.3 |
| c. | Ni(3), Mo(12) on alumina | 0.4 | 0.2 |
| d. | Ni(3), Ta(12) on alumina | 1.7 | 0.1 |
| e. | Ni(3), V(10) on alumina | 0.6 | 0.03 |
| f. | Ni(3), Th(12) on alumina | 0.2 | 0 |
| g. | Ni(3), W(12), Si(0.2) on alumina | 1.0 | 0.1 |
| h. | Ni(6), Mo(15), P(2) on silica-alumina | 4.4 | 0.9 |

[1]Approximate weight percent of metal in parenthesis, present in oxide form.

Example 9

An 18 ml stainless steel reactor was charged with 5 ml ethanol, 0.125 g sulphur, and 0.50 g of a catalyst comprising nickel, molybdenum, and phosphorous (approximately 6, 15, and 2 wt.%) oxides on silica-alumina. The reactor was sealed and pressurized to 900 psi with a mixture of 2 parts hydrogen and 1 part carbon monoxide, then heated with gentle shaking for 4.0 hours at 240°C. After cooling and releasing the pressure, analysis indicated ethyl propionate (1.2 mmol) and n-propanol (0.6 mmol) as the major carbonylation products along with an undetermined amount of ethyl ether.

Example 10

An 18 ml stainless steel reactor was charged with 5 ml of 1,3-propanediol, 0.125 g of sulphur, and 0.5 g of a catalyst comprising nickel, molybdenum, and phosphorous (approximately 6, 15, and 2 wt.%) oxides on silica-alumina. The reactor was sealed, pressurized with 900 psi of a mixture of two parts hydrogen and one part carbon monoxide, then heated with gentle shaking to 240°C for 4.0 hours. After cooling and releasing the pressure, gas chromatographic analysis indicated the presence of gamma-butyrolactone.

Example 11

Two 18 ml stainless steel reactors were each charged with 1 ml of methanol, 0.125 g sulphur, and 0.50 g of a catalyst comprising nickel, molybdenum, and phosphorous (approximately 6, 15, and 2 wt%) oxides on silica-alumina. Additionally one reactor was charged with 4 ml N-methylpyrrolidinone and the other with 4 ml of tetra-hydrofuran. The reactors were sealed and pressurized to 900 psi with a mixture of 2 parts hydrogen and 1 part carbon monoxide. The reactors were heated with gentle shaking for 4.0 hours at 240°C. Analysis of the products indicated 0.6 mmol methyl acetate and minor amounts of acetaldehyde and ethanol in the first, and 1.86 mmol of methyl acetate in the second as well as methyl ether and methyl sulphide in both.

Example 12

A stainless steel reactor tube was packed with 5.0 g of catalyst comprising nickel, molybdenum, and phosphorous (approximately 3, 13, and 2 wt.%) oxides on alumina. The catalyst was treated with 10% hydrogen sulphide in hydrogen at 325°C for 1 hour, then purged with nitrogen. The catalyst bed was maintained at 280°C as synthesis gas (comprising two parts hydrogen and one part carbon monoxide) and methanol were passed over the catalyst. The reactor pressure was maintained between 1720 and 1760 psi, the GHSV at 4000 hr.$^{-1}$ and LHSV at 2 hr.$^{-1}$. Analysis of the products collected over a 20.5 hour period indicated methyl acetate (79.2 mmol) and ethanol (102.2 mmol) as major carbonylation products as well as a lesser amount of acetic acid. Methyl ether was also formed.

Example 13

A 300 ml stainless steel autoclave was charged with 75 ml methanol and 5.0 g of a 10—12% nickel on gamma alumina catalyst (Girdler T-310) that had been treated with 10% hydrogen sulphide in hydrogen at 325°C. The reactor was sealed, pressurized with a mixture of one part hydrogen and two parts carbon

5

monoxide, and heated to 240°C. The pressure was maintained at 2150 to 2250 psi for 6 hours. After cooling and venting the gases, the liquid contents were examined by gas chromatography using 1,4-dioxan as an internal standard. In addition to methanol and methyl ether, 10.5 mmol methyl acetate, 1.0 mmol ethanol, and a small amount of methyl formate were produced.

Example 14
A 3% nickel on alumina catalyst was prepared by impregnating 50.0 g of alumina with 32 ml of a water solution containing 7.65 g of nickel nitrate hexahydrate. The catalyst was dried in a vacuum oven at approximately 100°C and then calcined at 475°C for 4 hours.

Example 15
An 18 ml stainless steel reactor was charged with 5 ml methanol, 0.125 g of sulphur, 0.10 ml 1,4-dioxan, and 0.50 g of a 3% nickel on alumina catalyst (described in Example 14). The reactor was sealed and pressurized to 900 psi of a mixture of two parts hydrogen and one part carbon monoxide. After heating for 4.0 hours at 240°C, gas chromatographic analysis of the products, using 1,4-dioxan as an internal standard, indicated the presence of 0.51 mmol methyl acetate as well as methyl ether, methyl sulphide, and unreacted methanol.

Example 16
An 18 ml stainless steel reactor was charged with 5 ml methanol, 0.10 ml 1,4-dioxan, 0.125 g sulphur, and 0.50 g of a 10—12% nickel on gamma alumina catalyst (Girdler T-310). The reactor was sealed and pressurized to 900 psi with a mixture of two parts hydrogen and one part carbon monoxide. The reactor was heated to and maintained at 240°C for 4.0 hours, then cooled and vented. The liquid portion was analyzed by gas chromatography using 1,4-dioxan as an internal standard. In addition to methyl ether and methyl sulphide, 1.8 mmol of methyl acetate was produced.

Example 17
The procedure of Example 16 was followed except that ethanol was substituted for methanol. The major carbonylation product was ethyl propionate (0.1 mmol) with a trace of n-propanol.

Example 18
An 18 ml stainless steel reactor was charged with 5 ml methanol, 0.1 ml 1,4-dioxan, 0.13 g sulphur, and 0.25 g of a catalyst comprising 40% nickel on a refractory support (Girdler G87). The reactor was pressurized to 900 psi with a mixture of two parts hydrogen and one part carbon monoxide, sealed, heated to 240°C and shaken for 4 hours. After cooling and venting the gases, gas chromatographic analysis indicated the presence of methyl acetate (2.82 mmol) and ethanol (0.05 mmol) as the major carbonylation products.

Example 19
An unsupported nickel sulphide catalyst was prepared *in situ* by combining nickel carbonate (52 mg), sulphur (24 mg) methanol (5 ml), and 1,4-dioxan (0.10 ml) in an 18 ml stainless steel reactor. The reactor was pressurized to 900 psi with a mixture of 2 parts hydrogen and 1 part carbon monoxide, sealed, and heated with gentle shaking at 240°C for 4 hours. After cooling and venting the gases, the contents were removed to yield a clear liquid and a black solid. Gas chromatographic analysis of the liquid using 1,4-dioxan as an internal standard indicated that methyl acetate (0.67 mmol) and ethanol (0.03 mmol) were the major carbonylation products.

**Claims**

1. A process for catalytically converting an alcohol into at least one oxygen-containing product having at least one more carbon atom than the starting alcohol, characterised in that an alcohol having from one to twenty carbon atoms is reacted with hydrogen and carbon monoxide at a temperature in the range from 150 to 350°C and a pressure in the range from 500 to 5,000 psig (34 to 345 bar) in the presence of an insoluble heterogeneous sulphided catalyst comprising nickel, or nickel in admixture with a co-catalyst selected from the elements of Group VB, Group VIB and the Actinide series of the Periodic Table, the nickel and co-catalyst when present being in sulphide form, to obtain the desired reaction product.

2. A process according to Claim 1, wherein the heterogeneous sulphided catalyst comprises nickel sulphide and molybdenum sulphide.

3. A process according to Claim 1 or 2, wherein the heterogeneous sulphided catalyst further comprises phosphorus sulphide.

4. A process according to Claim 1, wherein the heterogeneous sulphided catalyst comprises nickel sulphide alone.

5. A process according to Claim 1, 2, 3 or 4, wherein the sulphided catalyst is present on a support.

6. A process according to Claim 5, wherein the support is alumina or silica-alumina.

**0 099 665**

7. A process according to any preceding claim, wherein the alcohol is methanol and the reaction product obtained includes one or more of acetic acid, ethanol, acetaldehyde and methyl acetate.

**Patentansprüche**

1. Verfahren zur katalytischen Umwandlung eines Alkohols in wenigstens ein Sauerstoff-enthaltendes Produkt mit wenigstens einem zusätzlichen Kohlenstoffatom gegenüber dem Ausgangsalkohol, dadurch gekennzeichnet, daß ein Alkohol mit 1 bis 20 Kohlenstoffatomen mit Wasserstoff und Kohlenmonoxid bei einer Temperatur im Bereich von 150 bis 350°C sowie einem Druck im Bereich von 500 bis 5 000 psig (34 bis 345 bar) in Gegenwart eines unlöslichen heterogenen sulfidierten Katalysators aus Nickel oder Nickel in Mischung mit einem Co-Katalysator, ausgewählt aus den Elementen der Gruppe VB, VIB und Actinidenreihe des Periodischen Systems der Elemente, wobei das Nickel und der Co-Katalysator, falls vorhanden, in der Sulfidform vorliegen, zur Gewinnung des gewünschten Reaktionsprodukts umgesetzt wird.

2. Verfahren nach Anspruch 1, dadurch gekennzeichnet, daß der heterogene sulfidierte Katalysator aus Nickelsulfid und Molybdänsulfid besteht.

3. Verfahren nach Anspruch 1 oder 2, dadurch gekennzeichnet, daß der heterogene sulfidierte Katalysator außerdem Phosphorsulfid enthält.

4. Verfahren nach Anspruch 1, dadurch gekennzeichnet, daß der heterogene sulfidierte Katalysator aus Nickelsulfid allein besteht.

5. Verfahren nach Anspruch 1, 2, 3 oder 4, dadurch gekennzeichnet, daß der sulfidierte Katalysator auf einem Träger vorliegt.

6. Verfahren nach Anspruch 5, dadurch gekennzeichnet, daß der Träger aus Aluminiumoxid oder Siliziumdioxid/Aluminiumoxid besteht.

7. Verfahren nach einem der vorhergehenden Ansprüche, dadurch gekennzeichnet, daß der Alkohol aus Methanol besteht und das erhaltene Reaktionsprodukt einen oder mehrere der Bestandteile Essigsäure, Ethanol, Acetaldehyd und Methylacetat enthält.

**Revendications**

1. Procédé de transformation catalytique d'un alcool en au moins un produit contenant de l'oxygène, ayant au moins un atome de carbone de plus que l'alcool de départ, caractérisé en ce qu'un alcool ayant un à vingt atomes de carbone est amené à réagir avec l'hydrogène et l'oxyde de carbone à une température comprise dans l'intervalle de 150 à 350°C et sous une pression manométrique comprise dans l'intervalle de 500 à 5000 lb/in$^2$ (34 à 345 bars) en présence d'un catalyseur sulfuré hétérogène insoluble contenant du nickel, ou bien du nickel mélangé à un co-catalyseur choisi entre les éléments du groupe VB, du groupe VIB et de la série des Actinides du Tableau Périodique, le nickel et le co-catalyseur, lorsqu'il est présent, étant sous forme de sulfures, afin d'obtenir le produit de réaction désiré.

2. Procédé suivant la revendication 1, dans lequel le catalyseur sulfuré hétérogène comprend le sulfure de nickel et le sulfure de molybdène.

3. Procédé suivant la revendication 1 ou 2, dans lequel le catalyseur sulfuré hétérogène comprend en outre le sulfure de phosphore.

4. Procédé suivant la revendication 1, dans lequel le catalyseur sulfuré hétérogène comprend le sulfure de nickel seul.

5. Procédé suivant la revendication 1, 2, 3, ou 4, dans lequel le catalyseur sulfuré est présent sur un support.

6. Procédé suivant la revendication 5, dans lequel le support est l'alumine ou un mélange silice-alumine.

7. Procédé suivant l'une quelconque des revendications précédentes, dans lequel l'alcool est le méthanol et le produit de réaction obtenu comprend un ou plusieurs composés choisis entre l'acide acétique, l'éthanol, l'acétaldéhyde et l'acétate de méthyle.